Europäisches Patentamt

European Patent Office

Office européen des brevets

(11) Veröffentlichungsnummer : **0 163 837**
**B1**

## (12) EUROPÄISCHE PATENTSCHRIFT

(45) Veröffentlichungstag der Patentschrift :
20.01.88

(51) Int. Cl.⁴ : **C 07 C 118/02**

(21) Anmeldenummer : 85103502.2

(22) Anmeldetag : 25.03.85

(54) **Verfahren zur Herstellung von Polyisocyanaten.**

(30) Priorität : 07.04.84 DE 3413174

(43) Veröffentlichungstag der Anmeldung :
11.12.85 Patentblatt 85/50

(45) Bekanntmachung des Hinweises auf die Patenterteilung : 20.01.88 Patentblatt 88/03

(84) Benannte Vertragsstaaten :
BE DE FR GB IT NL

(56) Entgegenhaltungen :
DD-A-  144 559
DE-A- 3 129 270
ULLMANNS  ENYKLOPÄDIE  DER  TECHNISCHEN
CHEMIE "Hormone bis Keramik" 4. Auflage, Band 13
VERLAG CHEMIE, Weinheim, New York Seite 312
ULLMANNS  ENCYKLOPÄDIE  DER  TECHISCHEN
CHEMIE "Hormone bis Keramik" 4. Auflage, Band 13
VERLAG CHEMIE, Weinheim, New York Seiten 295-
299

(73) Patentinhaber : BAYER AG
Konzernverwaltung RP Patentabteilung
D-5090 Leverkusen 1 Bayerwerk (DE)

(72) Erfinder : Keggenhoff, Berthold, Dr.
Im Paradies 32
D-4150 Krefeld 29 (DE)
Erfinder : Ellendt, Günther, Dr.
Deswatinesstrasse 81
D-4150 Krefeld 1 (DE)
Erfinder : Petinaux, Marcel, Dr.
Flensburger Zeile 1 h
D-4150 Krefeld 1 (DE)
Erfinder : Mitschker, Alfred, Dr.
Am Gartenfeld 50
D-5068 Odenthal (DE)
Erfinder : Lange, Peter Michael, Dr.
Walter-Flex-Strasse 9
D-5090 Leverkusen 1 (DE)

**Beschreibung**

Die Erfindung betrifft ein neues Verfahren zur Herstellung von organischen Polyisocyanaten in Gegenwart von Lösungsmitteln und unter Wiederverwendung des eingesetzten Lösungsmittels, wobei man das Lösungsmittel vor seiner Wiederverwendung zumindest teilweise mit vernetzten Polymeren, bevorzugt Styrol-Divinylbenzol-Perlpolymerisaten behandelt, die als funktionelle Gruppen primäre oder sekundäre alkoholische Gruppen oder primäre oder sekundäre Aminogruppen besitzen.

Organische Polyisocyanate werden großtechnisch durch Phosgenierung der ihnen entsprechenden primären Polyamine hergestellt, wobei man die Phosgenierung in Gegenwart von inerten organischen Lösungsmitteln wie z. B. Chlorbenzol oder ortho-Dichlorbenzol durchführt (vgl. z. B. Ullmanns Encyclopädie der technischen Chemie, 4. Auflage (1977), Band 13, Seiten 350 ff.).

Bei der Herstellung der technisch wichtigen Polyisocyanate, insbesondere bei der Herstellung von Hexamethylendiisocyanat, Toluylendiisocyanaten und Polyisocyanaten der Diphenylmethanreihe durch Phosgenierung der entsprechenden Di- und Polyamine entstehen stets Spuren an Isocyanatgruppen aufweisenden Verbindungen (z. B. 6-Chlor-hexylisocyanat im Falle der Herstellung von Hexamethylendiisocyanat, Tolylisocyanat im Falle der Herstellung von Toluylendiisocyanaten und Phenylisocyanat im Falle der Herstellung von Polyisocyanaten der Diphenylmethanreihe durch Phosgenierung von Anilin/Formaldehyd-Kondensaten). Zur Verringerung des Gehalts der Verfahrensprodukte (Polyisocyanate) an derartigen, Isocyanatgruppen aufweisenden, die Qualität der Verfahrensprodukte äußerst negativ beeinflussenden Verbindungen war man bislang bestrebt, diese nach der Phosgenierungsreaktion zusammen mit dem Lösungsmittel destillativ aus dem Polyisocyanat zu entfernen und das Lösungsmittel seinerseits vor seiner Wiederverwendung durch eine aufwendige Kolonnendestillation von den Verunreinigungen zu befreien. Diese destillative Aufarbeitung des Lösungsmittels ist nur mit einem hohen Energie- und apparativen Aufwand möglich, wobei insbesondere diejenigen Verbindungen Schwierigkeiten bereiten, deren Siedepunkte in der Nähe der Siedepunkte der jeweiligen Lösungsmittel liegen.

Die DE-OS 3 129 270 beschreibt nun ein Verfahren zur Herstellung von Polyisocyanaten in Gegenwart von Lösungsmitteln, wobei das Lösungsmittel vor seiner Wiederverwendung dadurch von Spuren an Isocyanatgruppen aufweisenden Verbindungen befreit wird, daß man es mit Verbindungen mit gegenüber Isocyanatgruppen reaktionsfähigen Wasserstoffatomen, wie Alkoholen oder Aminen, behandelt, wodurch die leichtflüchtigen Isocyanate in Urethan- oder Harnstoffgruppen aufweisende Reaktionsprodukte überführt werden, und anschließend das behandelte Lösungsmittel destillativ von diesen Reaktionsprodukten abtrennt.

Wenn es auch wesentlich geringeren Aufwand bedeutet, die so erzeugten Reaktionsprodukte, die wesentlich höhere Siedepunkte besitzen als die Isocyanatspuren, destillativ abzutrennen, so ist es doch zur Ausschleusung dieser Nebenprodukte erforderlich, die gesamte bzw. die zur Zubereitung der Polyaminlösung benötigte Lösungsmittelmenge zu destillieren. Dies bedeutet wegen der hohen erforderlichen Lösungsmittelmengen einen großen Energieaufwand.

Unterläßt man jedoch eine destillative Abtrennung der nach o. g. DE-OS zunächst erzeugten Harnstoffe oder Urethane, so gelangen diese bei der Wiederverwendung des Lösungsmittels in den Phosgenierungsprozeß, wo sie sich auf vielfältige Weise weiter mit Phosgen sowie mit den neu erzeugten Isocyanaten umsetzen können und so in unterschiedlicher Weise die Polyisocyanatqualität bzw. die Ausbeute zu destilliertem Diisocyanat beeinträchtigen können.

Es war daher die der Erfindung zugrundeliegende Aufgabe, ein neues Verfahren zur Abtrennung von Isocyanatspuren aus dem beim Verfahren zur Polyisocyanatherstellung anfallenden Lösungsmittel zur Verfügung zu stellen, welches ohne Destillationsaufwand ein sowohl von Isocyanatspuren als auch von sonstigen Nebenprodukten freies Lösungsmittel zur Wiederverwendung im technisch üblichen Verfahren der Polyisocyanatherstellung ergibt.

Diese Aufgabe konnte überraschenderweise dadurch gelöst werden, daß man das von den Isocyanatspuren zu befreiende Lösungsmittel mit bestimmten Polymeren der nachstehend näher beschriebenen Art behandelt und anschließend mechanisch von diesen Polymeren abtrennt. Hierbei war insbesondere die Beobachtung überraschend, daß diese Polymeren als Festphase bei relativ kurzer Kontaktzeit mit dem Lösungsmittel die in sehr geringer Konzentration vorliegenden Isocyanate zu binden vermögen, wobei die Polymeren fast 50 % ihres Eigengewichts an Isocyanaten aufzunehmen in der Lage sind.

Gegenstand der Erfindung ist somit ein Verfahren zur Herstellung von Polyisocyanaten durch Umsetzung von (i) Lösungen der den Polyisocyanaten zugrundeliegenden Polyamine in einem inerten Lösungsmittel mit (ii) einer Lösung von Phosgen in einem inerten Lösungsmittel in einer ein- oder mehrstufigen Phosgenierungsreaktion, destillative Aufarbeitung des Reaktionsgemisches nach Beendigung der Phosgenierungsreaktion und Wiederverwendung des Lösungsmittels zur Herstellung von Aminlösung (i) und von Phosgenlösung (ii) nach Entfernung von Isocyanatspuren aus dem Lösungsmittel, wobei entweder die Gesamtmenge des wiedergewonnenen Lösungsmittels mit Verbindungen mit gegenüber Isocyanatgruppen reaktionsfähigen Wasserstoffatomen behandelt und anschließend von, durch Reaktion dieser Verbindungen mit gegenüber Isocyanatgruppen reaktionsfähigen Wasserstoffatomen mit im Lösungsmittel enthaltenen, Isocyanatgruppen aufweisenden Verbindungen gebildeten, Reaktionsprodukten befreit, oder zumindest ein Teil der zur Herstellung der Aminlösung (i) verwendeten Teilmenge des Lösungsmittels auf diese Weise behandelt wird, dadurch gekennzeichnet, daß man als Verbindungen mit gegenüber Isocyanatgruppen reaktionsfähigen Wasserstoffatomen vernetzte, in dem Lösungsmittel unlösliche Polymere verwendet, die als funktionelle Gruppen primäre oder sekundäre alkoholische Hydroxylgruppen oder primäre oder sekundäre aminische Aminogruppen aufweisen, und man das Lösungsmittel von den Reaktionsprodukten und überschüssigen Polymeren durch mechanische Trennung abtrennt.

Das erfindungsgemäße Prinzip der Lösungsmittelaufbereitung eignet sich insbesondere für ein mehrstufiges Verfahren zur Herstellung von Polyisocyanaten, welches aus folgenden Einzelstufen besteht :

a) Umsetzung von (i) Lösungen der den Polyisocyanaten zugrundeliegenden Polyamine in einem inerten Lösungsmittel mit (ii) einer Lösung von Phosgen in einem inerten Lösungsmittel in einer ein- oder mehrstufigen Phosgenierungsreaktion,

b) Abtrennung des überschüssigen Phosgens und des gebildeten Chlorwasserstoffs aus dem gemäß a) erhaltenen flüssigen Reaktionsgemisch;

c) Abtrennung des Lösungsmittels zusammen mit leicht flüchtigen, NCO-Gruppen tragenden Verbindungen aus der gemäß b) erhaltenen Lösung durch eine Verdampfung und Gewinnung des gegebenenfalls einer weiteren destillativen Aufarbeitung zuzuführenden Verfahrensprodukts als Verdampfungsrückstand,

d) Gewinnung eines flüchtige, NCO-Gruppen tragende Verbindung enthaltenden Lösungsmittels durch Kondensation der gemäß c) anfallenden Dämpfe und Wiederverwendung eines Teils des Kondensats zur Herstellung von Aminlösung (i) und eines zweiten Teils des Kondensats zur Herstellung von Phosgenlösung (ii), wobei entweder

e) die Gesamtmenge des gemäß d) erhaltenen Kondensats, gegebenenfalls nach Entfernung von Phosgenresten als Kopfprodukt durch Andestillieren, jedoch ansonsten ohne weitere destillative Aufarbeitung mit Verbindungen mit gegenüber Isocyanatgruppen reaktionsfähigen Wasserstoffatomen behandelt und anschließend von, durch Reaktion dieser Verbindungen mit gegenüber Isocyanatgruppen reaktionsfähigen Wasserstoffatomen mit im Kondensat enthaltenen, Isocyanatgruppen aufweisenden Verbindungen gebildeten, Reaktionsprodukten befreit, oder

f) die aus gemäß d) erhaltenem Kondensat bestehende, zur Herstellung der Aminlösung (i) verwendete Teilmenge des Lösungsmittels, gegebenenfalls nach Entfernung von Phosgenresten als Kopfprodukt durch Andestillieren, jedoch ansonsten ohne weitere destillative Aufarbeitung mit Verbindungen mit gegenüber Isocyanatgruppen reaktionsfähigen Wasserstoffatomen behandelt und anschließend das so behandelte Lösungsmittel von, durch Reaktion dieser Verbindungen mit gegenüber Isocyanatgruppen reaktionsfähigen Wasserstoffatomen mit den Isocyanatgruppen aufweisenden Verbindungen gebildeten, Reaktionsprodukten befreit wird.

Die Phosgenierungsreaktion a) erfolgt in an sich bekannter Weise unter Verwendung von (i) Lösungen von Polyaminen in inerten Lösungsmitteln und Phosgenlösungen in inerten Lösungsmitteln, wobei auch beim erfindungsgemäßen Verfahren die Phosgenierungsreaktion ein- oder mehrstufig (beispielsweise Bildung von Suspensionen von Carbamidsäurechloriden in der Kälte und anschließende Überführung dieser Suspension in Polyisocyanatlösungen in der Hitze « Kalt/ Heißphosgenierung ») erfolgen kann. Für die Stufe a) des erfindungsgemäßen Verfahrens sind insbesondere die technisch wichtigen Polyamine wie beispielsweise Hexamethylendiamin, 2,4- und/ oder 2,6-Diaminotoluol, 2,4'- und 4,4'-Diaminodiphenylmethan sowie deren als « Polyamingemische der Diphenylmethanreihe » zu bezeichnenden Gemische mit höheren Homologen, wie sie durch Anilin/Formaldehyd-Kondensation in an sich bekannter Weise zugänglich sind, 1,5-Diaminonaphthalin, 1-Amino-3,3,5-trimethyl-5-aminomethyl-cyclohexan (Isophorondiamin), Tris-(isocyanatophenyl-)methan oder perhydrierte Diaminodiphenylmethane geeignet.

Bei der Durchführung des erfindungsgemäßen Verfahrens kommen die beispielhaft genannten Amine in Form vom 3- bis 40 gew.-%igen, vorzugsweise 5- bis 30 gew.-%igen Lösungen in inerten Lösungsmitteln zum Einsatz. Das bei der Phosgenierungsreaktion zum Einsatz gelangende Phosgen seinerseits kommt in Form von 10- bis 60 gew.%igen, vorzugsweise 25- bis 50 gew.-%igen Lösungen in inerten Lösungsmitteln zum Einsatz.

Geeignete inerte Lösungsmittel sowohl für das Polyamin als auch für das Phosgen sind beispielsweise Chlorbenzol oder ortho-Dichlorbenzol.

Nach der gemäß Verfahren des Standes der Technik durchgeführten Reaktionsstufe a) wird das überschüssige Phosgen und der gebildete Chlorwasserstoff beispielsweise durch Ausblasen mit Inertgas oder durch Andestillieren in der Reaktionsstufe b) entsprechend den bekannten Verfahren des Standes der Technik entfernt.

In der Stufe c) des erfindungsgemäßen Verfahrens wird das in Form einer Lösung vorliegende Phosgenierungsprodukt entweder durch einfach Abdampfen oder durch fraktionierte Destillation in eine, flüchtige NCO-Gruppen tragende Verbindungen enthaltende, Gasphase und eine im wesentlichen aus rohem Polyisocyanat bestehende Flüssigphase getrennt. Die hierbei anfallende flüssige Phase kann gewünschtenfalls zwecks Reindarstellung des Polyisocyanats in an sich bekannter Weise destillativ aufgearbeitet werden. Die Stufe c) des erfindungsgemäßen Verfahrens wird im allgemeinen bei einer Temperatur von 80 bis 220 °C, vorzugsweise 120 bis 190 °C bei einem Druck von 10 bis 4 000, vorzugsweise 200 bis 3 000 mbar durchgeführt.

Bei der Stufe d) des erfindungsgemäßen Verfahrens werden die gemäß c) anfallenden Dämpfe zu einem, flüchtige insbesondere Monoisocyanate enthaltenden Lösungsmittel-Kondensat kondensiert. Der Gehalt des Kondensats an den genannten Verbindungen (berechnet als NCO mit dem Molekulargewicht 42) kann bei 50 bis 5 000, insbesonders 100 bis 1 500 ppm (Gewicht) liegen.

Das Lösungsmittel wird nun zur erneuten Herstellung von Amin- und Phosgenlösungen wiederverwendet. Im Unterschied zu den bekannten Verfahren des Standes der Technik tritt jedoch an die Stelle weiterer destillativer Reinigungsoperationen, von einem gegebenenfalls durchzuführenden Andestillieren zwecks Entfernung von Phosgenresten als Kopfprodukt abgesehen, zwecks Beseitigung der Isocyanatgruppen aufweisenden Verbindungen eine der nachstehend näher beschriebenen erfindungsgemäßen Maßnahmen.

Gemäß der ersten Ausführungsform des erfindungsgemäßen Verfahrens wird das gemäß d) erhaltene Kondensat vor seiner Auftrennung in zur Herstellung von Aminlösung (i) und Phosgenlösung (ii) bestimmte Teilströme mit einem vernetzten, in dem Lösungsmittel unlöslichen Polymeren behandelt, welches gegenüber Isocyanatgruppen reaktionsfähige Gruppen, insbesondere primäre oder sekundäre alkoholische Hydroxylgruppen oder primäre oder sekundäre, an aliphatische Kohlenstoffatome gebundene, aminische Aminogruppen aufweist. Diese Polymere werden als Granulate oder, bevorzugt, in der Perlform eingesetzt. Es kann sich sowohl um Polykondensationsharze, Polymerisate bzw. Copolymerisate von (Meth-)Acrylsäurederivaten oder um Styrol-Divinylbenzol-Harze handeln, die herstellungsbedingt bereits gegenüber Isocyanatgruppen reaktionsfähige Gruppen der genannten Art aufweisen, oder in die im Anschluß an ihre Herstellung nach bekannten Methoden derartige Reaktivgruppen eingeführt worden sind.

Der Aufbau dieser Polymeren entspricht im allgemeinen dem entsprechender, bekannter Ionenaustauscherharze, wie sie z. B. zur Wasserentsalzung vielfach verwendet werden (vgl. z. B. Ullmanns Encyclopädie der technischen Chemie, 4. Auflage (1977) Band 13, s. 295 ff.). Dies bedeutet, daß die Herstellung der erfindungsgemäß geeigneten Polymeren und auch die Einführung der genannten funktionellen Gruppen nach den bekannten Methoden der Herstellung von Ionenaustauscherharzen erfolgen kann. So sind beispielsweise die Polykondensationsharze auf Basis von aliphatischen Polyaminen und Epichlorhydrin als erfindungsgemäß zu verwendende Polymere geeignet, die bereits herstellungsbedingt sekundäre Aminogruppen aufweisen. Erfindungsgemäß geeignete, Hydroxylgruppen aufweisende Perlcopolymerisate können beispielsweise durch die an sich bekannte Styrol-Divinylbenzol-Perlcopolymerisation unter Mitverwendung von Hydroxyalkylacrylaten oder -methacrylaten erhalten werden. Besonders bevorzugt sind jedoch Styrol-Divinylbenzol-Perlcopolymerisate mit einem Divinylbenzol-Anteil von mindestens 3, vorzugsweise von 5 bis 15 Gew.-%, in welche im Anschluß an ihre Herstellung primäre oder sekundäre, vorzugsweise primäre, an aliphatische Kohlenstoffatome gebundene aminische Aminogruppen eingeführt worden sind. So sind beispielsweise Styrol-Divinylbenzol-Perlcopolymerisate mit eingebauten Aminomethylgruppen besonders gut geeignet. Die Einführung derartiger Aminomethylgruppen in die Copolymerisate ist in der obengenannten Literaturstelle « Ullmann » auf Seite 302 beschrieben. Sekundäre Aminogruppen können in die Copolymerisate beispielsweise durch Umsetzung der entsprechenden chlormethylierten Zwischenprodukte mit primären Aminen erhalten werden. Besonders bevorzugt werden Perlpolymerisate bzw. -copolymerisate der genannten Art mit makroporöser Struktur eingesetzt. Der Gehalt der erfindungsgemäß zum Einsatz gelangenden Polymeren an gegenüber Isocyanatgruppen reaktionsfähigen Gruppen liegt im allgemeinen bei 1 bis 4, vorzugsweise 2 bis 3 Val/Liter an Lösungsmittel-gesättigtem, oftmals durch das Lösungsmittel gequollenem Polymeren. Die mittlere Teilchengröße der lösungsmittelfreien, in der Granulat- und vorzugsweise in der Perlform eingesetzten Polymeren liegt im allgemeinen bei 0,1 bis 5, vorzugsweise 0,3 bis 2 mm. Die Harze haben im allgemeinen ein Bindungsvermögen von 100 bis 400 g Isocyanat pro Liter an Lösungsmittel-gesättigtem Harz. Demzufolge ist 1 Liter Harz in der Lage, z. B. bis zu 4 t Lösungsmittel mit einem Isocyanatgehalt von 100 ppm (Gewicht) zu reinigen. Die jeweils erforderliche Harzmenge kann bei vorgegebener Lösungsmittelmenge mit einem vorgegebenen Gehalt an Verunreinigungen unter Beachtung dieser Daten und — insbesondere bei kontinuierlicher Arbeitsweise — unter Beachtung der obengemachten Ausführungen bezüglich der Kontaktzeit in einem kurzen Vorversuch zuverlässig ermittelt werden.

Die erfindungsgemäße Behandlung des gemäß d) erhaltenen Kondensats mit den erfindungsgemäß verwendeten Polymeren kann diskontinuierlich oder, bevorzugt, kontinuierlich erfolgen, wobei die für die Wasserbehandlung mit Ionenaustauschern entwickelte Technologie verwendet werden kann. Speziell kann die Behandlung in Festbett- oder Wirbelbettreaktoren oder in Rührkesselkaskaden erfolgen, wobei bevorzugt Wirbelbettreaktoren eingesetzt werden.

Die Behandlung kann ein- oder mehrstufig erfolgen. Sie erfolgt im allgemeinen in einem Temperaturbereich von 20 bis 180 °C, bevorzugt bei 50 bis 130 °C bei Normaldruck oder einem Überdruck bis 5 bar.

Dabei ist eine hinreichende Kontaktzeit mit dem Polymeren erforderlich, die, in Abhängigkeit von der Behandlungstemperatur, in Vorversuchen leicht ermittelt werden kann. So wurden beispielsweise Kontaktzeiten von 5 bis 15 Min. als ausreichend gefunden.

Nach der Behandlung ist eine mechanische Abtrennung des Polymeren, z. B. durch Dekantieren oder Filtrieren, entsprechend technisch üblicher Ionenaustauschertechnologie, vorzusehen.

Während dieser Behandlung kann das Polymere ca. 20 bis 50 % seines Gewichts an Isocyanat aufnehmen, ohne daß seine Struktur zerstört wird.

Das von überschüssigem Polymeren und von den Isocyanatgruppen aufweisenden Verbindungen in Form von deren Reaktionsprodukten mit den Polymeren befreite Lösungsmittel wird dann erneut zur Herstellung von Aminlösung (i) und Phosgenlösung (ii) eingesetzt. Auf diese Weise wird erreicht, daß bei der Durchführung des erfindungsgemäßen Verfahrens gemäß erster Ausführungsform Polyisocyanate mit einem stark reduzierten Gehalt an leichtflüchtigen Isocyanatkomponenten entstehen. Gleichzeitig wird der Energie- und apparative Aufwand bezüglich der durchzuführenden Destillationsschritte ganz wesentlich reduziert, ohne daß mit dem Lösungsmittel Nebenprodukte in die Polyisocyanate eingetragen werden.

Gemäß 2. Ausführungsform des erfindungsgemäßen Verfahrens wird das gemäß d) erhaltene Lösungsmittelkondensat entsprechend den Mengenströmen der Aminlösung (i) und Phosgenlösung (ii) in 2 Teilströme aufgeteilt und als Lösungsmittel zur Herstellung dieser Lösungen wiederverwendet. Das Gewichtsverhältnis dieser Lösungsmittelströme zur Herstellung der Lösungen (i) bzw. (ii) liegt bei beiden Ausführungsformen des erfindungsgemäßen · Verfahrens zwischen 10 : 1 und 1 : 5.

Gemäß 2. Ausführungsform des erfindungsgemäßen Verfahrens wird nun der zur Herstellung der Phosgenlösung (ii) verwendete Teilstrom des gemäß d) erhaltenen Lösungsmittelkondensats unbehandelt eingesetzt und lediglich der zur Herstellung der Aminlösung (i) eingesetzte Teilstrom entsprechend den obengemachten Ausführungen einer erfindungsgemäßen Behandlung unterzogen. Gemäß einer Variante dieser zweiten Ausführungsform des erfindungsgemäßen Verfahrens wird nur ein Teil von mindestens 10 Gew.-%, vorzugsweise mindestens 50 Gew.-% des zur Herstellung der Aminlösung (i) eingesetzten Teilstroms der erfindungsgemäßen Behandlung unterzogen. Hierdurch wird ebenfalls erreicht, daß aufgrund der fortlaufenden Bindung eines Teils der Isocyanatgruppen aufweisenden Verbindungen des kontinuierlich im Kreislauf zurückgeführten Lösungsmittels an das Polymere der Gesamtgehalt des Systems an derartigen Isocyanatverbindungen ein tolerierbares Maximum nicht übersteigt.

Die nachfolgenden Beispiele dienen zur weiteren Erläuterung des erfindungsgemäßen Verfahrens. Alle Angaben in « ppm » beziehen sich auf Gewichtsverhältnisse.

### Beispiel 1

In einem 1 l-Rührkolben werden 30 ml eines wasserfreien, in Chlorbenzol gequollenen, makroporösen Styrol-Divinylbenzol-Perlpolymerisats (Gewichtsverhältnis Styrol : Divinylbenzol = 11,5 : 1) mit einem Gehalt an 2,4 Val/l an Aminomethylgruppen (bezogen auf gequollenes Polymerisat) vorgelegt. Die mittlere Teilchengröße der Polymerisatperlen liegt zu über 80 Gew.-% zwischen 0,5 und 1,3 mm.

Dem Polymerisat werden 500 ml, 100 °C warmes Chlorbenzol mit einem Gehalt an Phenylisocyanat von 1 030 ppm zugegeben. Diese Lösung entsprach einem Lösungsmittelkondensat, wie es durch Verdampfen und anschließendes Kondensieren des Lösungsmittels aus dem Produkt einer technischen Phosgenierung von Anilin/Formaldehyd-Kondensaten anfällt, und das vor dem Verdampfen des Lösungsmittels von Phosgen und Chlorwasserstoff befreit wird. Die Lösung wurde bei 100 °C während 10 Minuten mit dem Perlpolymerisat verrührt. Anschließend wurde der Rührer abgestellt, worauf sich das Perlpolymerisat rasch absetzte, und das überstehende Lösungsmittel abdekantiert. Es hatte einen Phenylisocyanatgehalt von 20 ppm. Darauf wurde weiteres Chlorbenzol mit 1 030 ppm Phenylisocyanat in gleicher Weise in Portionen von 500 ml mit dem im Kolben verbleibenden Perlpolymerisat behandelt. Insgesamt wurden 7 Portionen (= 3,5 l) behandelt, wobei das behandelte Lösungsmittel einen Phenylisocyanatgehalt von 20 ppm besaß. Bei der achten Portion betrug der Phenylisocyanatgehalt nach der Behandlung 40 ppm, bei der neunten Portion 170 ppm und bei der zehnten Portion 240 ppm. Danach wurde der Versuch beendet. Insgesamt wurden also mit 30 ml Perlpolymerisat 5 l Chlorbenzol gereinigt, wobei das Perlpolymerisat zu diesem Zeitpunkt 5,7 g Phenylisocyanat aufgenommen hatte. Die Perlstruktur des Harzes war danach praktisch unverändert. Das behandelte Chlorbenzol enthielt außer den Restmengen Phenylisocyanat keine weiteren Nebenprodukte.

### Beispiel 2

In einer zweistufigen Rührkaskade wurde in jeden Rührkolben 50 ml des in Beispiel 1 genannten Perlpolymerisats gegeben. Am Überlauf von jedem Rührkolben waren Drahtsiebe angeordnet, die das Perlpolymerisat in dem jeweiligen Rührkolben festhielten. Jeder Rührkolben nahm bis zum Überlauf 250 ml Flüssigkeit auf.

Dann wurde Chlorbenzol mit einem Gehalt von 550 ppm Phenylisocyanat und einer Temperatur von 80 °C in die Kaskade gegeben. Der Durchsatz durch die Kaskade betrug 2,5 l/Std. Das aus der Kaskade ablaufende Chlorbenzol hatte anfänglich einen Gehalt an Phenylisocyanat von 20 ppm, der langsam anstieg, bis nach Durchsatz von 27 l ein Gehalt von 100 ppm Phenylisocyanat erreicht war. Danach wurde das Perlpolymerisat der ersten Stufe der Kaskade entfernt, das Perlpolymerisat der zweiten Stufe zur weiteren Aufsättigung in die erste Stufe gegeben und in die zweite Stufe 50 ml frisches Perlpolymerisat gegeben. Danach wurde in der oben beschriebenen Weise erneut die gleiche Chlorbenzollösung behandelt, wobei nach Durchsatz von 18 l der Phenylisocyanatge-

halt im Ablauf von 20 ppm auf 100 ppm anstieg.

Aus diesem Versuch berechnet sich eine Aufnahmefähigkeit des Perlpolymerisats von 10,9 g Phenylisocyanat auf 50 ml bzw. 218 g Phenylisocyanat/Liter Chlorbenzol-gequollenes Perlpolymerisat.

Das behandelte Chlorbenzol enthielt außer den Restmengen Phenylisocyanat keine weiteren Nebenprodukte. Die Perlstruktur des Harzes wurde während der Phenylisocyanataufnahme praktisch nicht verändert.

## Patentansprüche

1. Verfahren zur Herstellung von Polyisocyanaten durch Umsetzung von (i) Lösungen der den Polyisocyanaten zugrundeliegenden Polyamine in einem inerten Lösungsmittel mit (ii) einer Lösung von Phosgen in einem inerten Lösungsmittel in einer ein- oder mehrstufigen Phosgenierungsreaktion, destillative Aufarbeitung des Reaktionsgemisches nach Beendigung der Phosgenierungsreaktion und Wiederverwendung des Lösungsmittels zur Herstellung von Aminlösung (i) und von Phosgenlösung (ii) nach Entfernung von Isocyanatspuren aus dem Lösungsmittel, wobei entweder die Gesamtmenge des wiedergewonnenen Lösungsmittels mit Verbindungen mit gegenüber Isocyanatgruppen reaktionsfähigen Wasserstoffatomen behandelt und anschließend von, durch Reaktion dieser Verbindungen mit gegenüber Isocyanatgruppen reaktionsfähigen Wasserstoffatomen mit im Lösungsmittel enthaltenen, Isocyanatgruppen aufweisenden Verbindungen gebildeten, Reaktionsprodukten befreit, oder zumindest ein Teil der zur Herstellung der Aminlösung (i) verwendeten Teilmenge des Lösungsmittels auf diese Weise behandelt wird, dadurch gekennzeichnet, daß man als Verbindungen mit gegenüber Isocyanatgruppen reaktionsfähigen Wasserstoffatomen vernetzte, in dem Lösungsmittel unlösliche Polymere verwendet, die als funktionelle Gruppen primäre oder sekundäre alkoholische Hydroxylgruppen oder primäre oder sekundäre aminische Aminogruppen aufweisen, und man das Lösungsmittel von den Reaktionsprodukten und überschüssigen Polymeren durch mechanische Trennung abtrennt.

2. Verfahren zur Herstellung von Polyisocyanaten gemäß Anspruch 1 durch

a) Umsetzung von (i) Lösungen der den Polyisocyanaten zugrundeliegenden Polyamine in einem inerten Lösungsmittel mit (ii) einer Lösung von Phosgen in einem inerten Lösungsmittel in einer ein- oder mehrstufigen Phosgenierungsreaktion,

b) Abtrennung des überschüssigen Phosgens und des gebildeten Chlorwasserstoffs aus dem gemäß a) erhaltenen flüssigen Reaktionsgemisch,

c) Abtrennung des Lösungsmittels zusammen mit leicht flüchtigen, NCO-Gruppen tragenden Verbindungen aus der gemäß b) erhaltenen Lösung durch eine Verdampfung und Gewinnung des gegebenenfalls einer weiteren destillativen Aufarbeitung zuzuführenden Verfahrensprodukts als Verdampfungsrückstand,

d) Gewinnung eines flüchtige, NCO-Gruppen tragenden Verbindung enthaltenden Lösungsmittels durch Kondensation der gemäß c) anfallenden Dämpfe und Wiederverwendung eines Teils des Kondensats zur Herstellung von Aminlösung (i) und eines zweiten Teils des Kondensats zur Herstellung von Phosgenlösung (ii), wobei entweder

e) die Gesamtmenge des gemäß d) erhaltenen Kondensats, gegebenenfalls nach Entfernung von Phosgenresten als Kopfprodukt durch Andestillieren, jedoch ansonsten ohne weitere destillative Aufarbeitung mit Verbindungen mit gegenüber Isocyanatgruppen reaktionsfähigen Wasserstoffatomen behandelt und anschließend von, durch Reaktion dieser Verbindungen mit gegenüber Isocyanatgruppen reaktionsfähigen Wasserstoffatomen mit im Kondensat enthaltenen, Isocyanatgruppen aufweisenden Verbindungen gebildeten, Reaktionsprodukten befreit, oder

f) die aus gemäß d) erhaltenem Kondensat bestehende, zur Herstellung der Aminlösung (i) verwendete Teilmenge des Lösungsmittels, gegebenenfalls nach Entfernung von Phosgenresten als Kopfprodukt durch Andestillieren, jedoch ansonsten ohne weitere destillative Aufarbeitung mit Verbindungen mit gegenüber Isocyanatgruppen reaktionsfähigen Wasserstoffatomen behandelt und anschließend das so behandelte Lösungsmittel von, durch Reaktion dieser Verbindungen mit gegenüber Isocyanatgruppen reaktionsfähigen Wasserstoffatomen mit den Isocyanatgruppen aufweisenden Verbindungen gebildeten, Reaktionsprodukten befreit wird, dadurch gekennzeichnet, daß man als Verbindungen mit gegenüber Isocyanatgruppen reaktionsfähigen Wasserstoffatomen vernetzte, in dem Lösungsmittel unlösliche Polymere verwendet, die als funktionelle Gruppen primäre oder sekundäre alkoholische Hydroxylgruppen oder primäre oder sekundäre aminische Aminogruppen aufweisen, und man das Lösungsmittel von den Reaktionsprodukten und überschüssigen Polymeren durch mechanische Trennung abtrennt.

3. Verfahren gemäß Anspruch 1 und 2, dadurch gekennzeichnet, daß man als Lösungsmittel Chlorbenzol oder o-Dichlorbenzol verwendet.

4. Verfahren gemäß Anspruch 1 bis 3, dadurch gekennzeichnet, daß man als vernetzte, im Lösungsmittel unlösliche Polymere in granulierter Form oder in Perlform vorliegende Polykondensationsharze oder Polymerisate verwendet.

5. Verfahren gemäß Anspruch 1 bis 4, dadurch gekennzeichnet, daß man als Polymere Perlpolymerisate oder Perlcopolymerisate von (Meth-)Acrylsäurederivaten oder Styrol/Divinylbenzol-Copolymerisate verwendet.

6. Verfahren gemäß Ansprüchen 1 bis 5, dadurch gekennzeichnet, daß man als Polymere makroporöse Styrol-Divinylbenzol-Perlpolymerisate mit primären und/oder sekundären, an aliphatische Kohlenstoffatome gebundenen Aminogruppen verwendet.

7. Ausführungsform der Verfahren gemäß Ansprüchen 2 bis 6, dadurch gekennzeichnet, daß man nur einen Teil von mindestens 10 Gewichtsprozent des gemäß d) erhaltenen und zur Herstellung der Aminlösung (i) verwendeten Lösungsmittels der in Anspruch 1 genannten Behandlung unterzieht.

**Claims**

1. Process for the preparation of polyisocyanates by the reaction of (i) solutions of the polyamines on which the polyisocyanates are based in an inert solvent with (ii) a solution of phosgene in an inert solvent in a single-stage or multi-stage phosgenation reaction, working up of the reaction mixture by distillation after termination of the phosgenation reaction and re-use of the solvent for the preparation of the amine solution (i) and phosgene solution (ii) after the removal of traces of isocyanate from the solvent, either the total quantity of the recovered solvent being treated with compounds containing hydrogen atoms which are reactive towards isocyanate groups and subsequently freed from reaction products formed by the reaction of these compounds containing hydrogen atoms which are reactive towards isocyanate groups with isocyanate-group-containing compounds contained in the solvent or at least a portion of that partial quantity of solvent that is used for preparing the amine solution (i) being treated in this manner, characterised in that cross-linked polymers which are insoluble in the solvent and contain primary or secondary alcoholic hydroxyl groups or primary or secondary aminic amino groups as functional groups are used as the compounds containing hydrogen atoms which are reactive towards isocyanate groups and the solvent is separated from the reaction products and excess polymers by mechanical separation.

2. Process for the preparation of polyisocyanates according to Claim 1, by

a) the reaction of (i) solutions in an inert solvent of the polyamines on which the polyisocyanates are based with (ii) a solution of phosgene in an inert solvent in a single-stage or multi-stage phosgenation reaction,

b) separation of the excess phosgene and of the hydrogen chloride formed from the liquid reaction mixture obtained according to a),

c) separation of the solvent together with readily volatile compounds carrying NCO groups from the solution obtained according to b) by evaporation and recovery of the product of the process, which is optionally to be subjected to further working up by distillation, as evaporation residue,

d) recovery of a solvent containing volatile compound carrying NCO groups by condensation of the vapours obtained according to c) and re-use of part of the condensate for the preparation of amine solution (i) and of a second part of the condensate for the preparation of phosgene solution (ii), either

e) the total quantity of the condensate obtained according to d) being treated with compounds containing hydrogen atoms which are reactive towards isocyanate groups, optionally after removal of residues of phosgene as head product by brief distillation but otherwise without any further working up by distillation, the condensate being subsequently freed from reaction products formed by the reaction of these compounds containing hydrogen atoms which are reactive towards isocyanate groups with isocyanate-group-containing compounds contained in the condensate, or

f) that partial quantity of the solvent consisting of condensate obtained according to d) which is used for preparation of the amine solution (i) being treated with compounds containing hydrogen atoms which are reactive towards isocyanate groups, optionally after removal of residues of phosgene as head product by brief distillation but otherwise without any further working up by distillation, and the solvent thus treated then being freed from reaction products formed by the reaction of these compounds containing hydrogen atoms which are reactive towards isocyanate groups with the compounds containing isocyanate groups, characterised in that cross-linked polymers which are insoluble in the solvent and contain primary or secondary alcoholic hydroxyl groups or primary or secondary aminic amino groups as functional groups are used as the compounds containing hydrogen atoms which are reactive towards isocyanate groups and the solvent is separated from the reaction products and excess polymers by mechanical separation.

3. Process according to Claim 1 and 2, characterised in that the solvent used is chlorobenzene or o-dichlorobenzene.

4. Process according to Claim 1 to 3, characterised in that polycondensation resins or polymers present in granulated form or in bead form are used as the cross-linked polymers which are insoluble in the solvent.

5. Process according to Claim 1 to 4, characterised in that the polymers used are bead polymers or bead copolymers of (meth)acrylic acid derivatives or styrene/divinyl benzene copolymers.

6. Process according to Claims 1 to 5, characterised in that the polymers used are macroporous styrene/divinyl benzene bead polymers having primary and/or secondary amino groups attached to aliphatic carbon atoms.

7. Embodiment of the processes according to Claims 2 to 6, characterised in that only a portion, amounting to at least 10 per cent by weight, of the solvent obtained according to d) and used for the preparation of amine solution (i) is subjected to the treatment mentioned in Claim 1.

**Revendications**

1. Procédé de préparation de polyisocyanates par réaction (i) de solutions des polyamines à la base des polyisocyanates, dans un solvant inerte, avec (ii) une solution de phosgène dans un solvant inerte au cours d'une réaction de phosgénation à une ou plusieurs étapes, en traitant le mélange réactionnel par distillation au terme de la réaction de phosgénation et en récupérant le solvant pour la préparation de la solution d'amines (i) et de la solution de phosgène (ii) après élimination des traces d'isocyanate hors du solvant, la quantité totale du solvant récupéré étant traitée avec des composés comportant des atomes d'hydrogène réactifs vis-à-vis des groupes isocyanate, pour être ensuite libérée des produits réactionnels formés par la réaction de ces composés comportant des atomes d'hydrogène réactifs vis-à-vis des groupes isocyanate, avec des composés comportant des groupes isocyanate et contenus dans le solvant, ou en traitant de la sorte au moins une partie de la quantité partielle de solvant qui a été utilisée pour la préparation de la solution d'amines (i), caractérisé en ce que, comme composés comportant des atomes d'hydrogène réactifs vis-à-vis des groupes isocyanate, on utilise des polymères réticulés insolubles dans le solvant et comportant comme groupes fonctionnels, des groupes hydroxyle alcooliques primaires ou secondaires, ou des groupes amino aminiques primaires ou secondaires et l'on sépare le solvant des produits réactionnels et des polymères en excès par séparation mécanique.

2. Procédé de préparation de polyisocyanates selon la revendication 1, dans lequel :

a) on fait réagir (i) des solutions des polyamines à la base des polyisocyanates, dans un solvant inerte, avec (ii) une solution de phosgène dans un solvant inerte, au cours d'une réaction de phosgénation en une ou plusieurs étapes,

b) on sépare le phosgène en excès et l'acide chlorhydrique formé hors du mélange réactionnel liquide obtenu selon a),

c) on sépare le solvant conjointement avec des composés très volatils comportant des groupes NCO, hors de la solution obtenue selon b) moyennant une évaporation et l'obtention, comme résidu d'évaporation, du produit du procédé devant éventuellement être acheminé à un traitement complémentaire par distillation,

d) on forme un solvant volatil contenant un composé comportant des groupes NCO, par condensation des vapeurs obtenues selon c) et on réutilise une partie du condensat pour préparer la solution d'amines (i), ainsi qu'une deuxième partie du condensat pour préparer la solution de phosgène (ii), et soit

e) on traite la quantité totale du condensat obtenu selon d), éventuellement après élimination des résidus de phosgène comme produit de tête, moyennant une approche de distillation mais, par ailleurs, sans traitement complémentaire par distillation, avec des composés comportant des atomes d'hydrogène réactifs vis-à-vis des groupes isocyanate, puis on la libère des produits réactionnels formés par la réaction de ces composés comportant des atomes d'hydrogène réactifs vis-à-vis des groupes isocyanate, avec des composés comportant des groupes isocyanate et contenus dans le condensat, soit

f) on traite la quantité partielle de solvant, qui est constituée du condensat obtenu selon d) et qui a été utilisée pour la préparation de la solution d'amines (i), éventuellement après élimination des résidus de phosgène comme produit de tête, moyennant une approche de distillation mais, par ailleurs, sans traitement complémentaire par distillation, avec des composés comportant des atomes d'hydrogène réactifs vis-à-vis des groupes isocyanate, puis on libère le solvant ainsi traité des produits réactionnels formés par la réaction de ces composés comportant des atomes d'hydrogène réactifs vis-à-vis des groupes isocyanate, avec les composés comportant des groupes isocyanate, caractérisé en ce que, comme composés comportant des atomes d'hydrogène réactifs vis-à-vis des groupes isocyanate, on utilise des polymères réticulés insolubles dans le solvant et comportant, comme groupes fonctionnels, des groupes hydroxyle alcooliques primaires ou secondaires, ou des groupes amino aminiques primaires ou secondaires et l'on sépare le solvant des produits réactionnels et des polymères en excès par séparation mécanique.

3. Procédé selon les revendications 1 et 2, caractérisé en ce que, comme solvant, on utilise le chlorobenzène ou l'o-dichlorobenzène.

4. Procédé selon les revendications 1 à 3, caractérisé en ce que, comme polymères réticulés insolubles dans le solvant, on utilise des polymères ou des résines de polycondensation se présentant sous forme de granulés ou de perles.

5. Procédé selon les revendications 1 à 4, caractérisé en ce que, comme polymères, on utilise des polymères en perles ou des copolymères en perles de dérivés d'acide (méth)acrylique ou des copolymères de styrène/divinylbenzène.

6. Procédé selon les revendications 1 à 5, caractérisé en ce que, comme polymères, on utilise des polymères en perles macroporeux de styrène/divinylbenzène comportant des groupes amino primaires et/ou secondaires liés à des atomes de carbone aliphatiques.

7. Forme de réalisation des procédés selon les revendications 2 à 6, caractérisée en ce qu'on soumet, au traitement mentionné dans la revendication 1, uniquement une partie d'au moins 10 % en poids du solvant obtenu selon d) et utilisé pour la préparation de la solution d'amines (i).